# FASCICULE DE BREVET EUROPEEN

(11) **EP 2 182 876 B1**
(45) Date de publication et mention de la délivrance du brevet: **16.01.2013**
(21) Numéro de dépôt: 08845471.5
(22) Date de dépôt: 05.08.2008
(51) Int. Cl.: A61B 18/20, A61N 5/067

(54) **PIECE A MAIN REFROIDIE POUR LE TRAITEMENT DE LA PEAU PAR RAYONNEMENT LUMINEUX**
GEKÜHLTES WERKZEUG ZUR BEHANDLUNG DER HAUT MIT SICHTBARER STRAHLUNG
COOLED HANDPIECE FOR TREATING THE SKIN WITH VISIBLE RADIATION

(30) Priorité: 16.08.2007 FR 0705859
(43) Date de publication de la demande: 12.05.2010
(73) Titulaire: LSO MEDICAL, Loos (FR)
(72) Inventeur: ZEMMOURI, Jaouad, F-59242 Genech (FR); RINGOT, Jean, F-59370 Mons-En-Baroeul (FR); ZEMMOURI, Mohammed, F-59260 Hellemmes (FR)
(74) Mandataire: Matkowska, Franck
(86) Numéro de dépôt international: PCT/FR2008/001170
(87) Numéro de publication internationale: WO 2009/056690

(56) Documents cités:
- EP-A- 0 827 716
- WO-A-00/54685
- WO-A-99/52594
- US-A- 6 059 820
- US-A1- 2002 120 315

## Description

### Domaine technique

La présente invention concerne une pièce à main destinée à irradier une zone de traitement, par exemple la peau d'un patient, à l'aide d'une source d'énergie lumineuse et notamment une source laser. Elle trouve principalement son application dans le domaine médical de la dermatologie et plus particulièrement pour des traitements vasculaires, dépilatoires ou pigmentaires.

### Art antérieur

Les pièces à main sont utilisées de manière courante dans le domaine médical de la dermatologie et plus particulièrement dans le cas de traitements vasculaires, dépilatoires et pigmentaires. De telles pièces à main sont généralement reliées par une fibre optique à une source d'énergie lumineuse, et en particulier une source laser, qui permet de générer un faisceau lumineux à focaliser sur une zone de traitement. A cet effet, la pièce à main est pourvue d'une fenêtre, le plus souvent réalisée en saphir, apte à laisser passer le faisceau lumineux irradiant. Le saphir représente un réel avantage car outre sa bonne efficacité optique il présente une bonne conductivité thermique nécessaire pour son refroidissement.

En effet, dans le domaine de la dermatologie et des traitements de la peau d'un patient par une source lumineuse tel qu'un laser, il est souvent nécessaire de refroidir la peau du patient ou l'élément au contact de la peau du patient. Ce refroidissement est important car il permet d'une part de prévenir les risques de brûlures causées par l'irradiation laser sur la peau du patient et d'autre part d'atténuer la douleur dans la zone de traitement au moment des impacts du tir laser.

Ce refroidissement de la peau ou de l'élément au contact de la peau engendre cependant un premier inconvénient lié à l'apparition de condensation sur la fenêtre. Le phénomène de condensation est notamment mis en évidence dans la publication « Effect of ambiant humidity on light transmittance through skin phantoms during cryogen spray cooling » de Julio C Ramirez-San-Juan, Bernard Choi, Walfre Franco, J.Stuart Nelson et Guillermo Aguilar, Physics in Medicine and Biology, 51 (2006) pages 113-120. La condensation est néfaste pour la bonne réalisation du traitement car elle atténue la transmittance du faisceau lumineux et réduit ainsi la puissance du tir. De plus, la condensation nuit également à la bonne visibilité de la zone de traitement et du tir dans le cas de pièce à main munie d'un dispositif de visualisation telle qu'une caméra.

Un deuxième inconvénient des pièces à main munies de dispositif de refroidissement réside dans leur compacité et leur facilité d'utilisation. Les dispositifs non intégrés consistant à appliquer un gel refroidissant sur la peau du patient sont peu pratiques et les pièces à main pourvues d'un spray cryogénique intégré sont encombrantes.

Pour ces raisons, une pièce à main comprenant une fenêtre en saphir associée à un dispositif de refroidissement par effet Peltier s'avère être une excellente solution, car elle est compacte comparativement au dispositif utilisant un spray cryogénique intégré à la pièce à main, et est plus facile d'utilisation par rapport aux dispositifs non intégrés qui consistent à appliquer un gel froid sur la peau préalablement au tir laser.

Le refroidissement au moyen d'un dispositif thermoélectrique à effet Peltier agit à la manière d'une pompe à chaleur. Plus précisément, l'effet Peltier est un phénomène physique de déplacement de chaleur d'une source froide vers une source chaude en présence d'un courant électrique. Le dispositif fonctionne à la manière d'une pompe à chaleur, la face froide se refroidit alors que la face chaude se réchauffe.

Une telle pièce à main comprenant un dispositif de refroidissement thermoélectrique est par exemple décrite dans la demande de brevet Américain US 2002/0120315. Elle comprend notamment une première fenêtre en saphir destinée à être au contact de la peau du patient, une unité de refroidissement par effet Peltier associée à la première fenêtre, et une seconde fenêtre en verre transparent recouverte d'un couvercle ajouré en aluminium. L'unité de refroidissement est assujettie à des moyens de contrôle de la température qui sont en relation avec des capteurs de température. Ces moyens de contrôle permettent de fixer une première température pour limiter la formation de condensation sur la fenêtre en saphir et une seconde température, inférieure à la première température, pour l'utilisation de la pièce à main au contact de la peau. La première fenêtre et la seconde fenêtre sont séparées par le support et des isolants thermiques, dont l'un d'entre eux est un espace clos et l'autre une résine. Egalement, la source chaude du dispositif de refroidissement thermoélectrique est située contre une plaque de refroidissement dans laquelle circule un liquide de refroidissement.

Ce dispositif de refroidissement visant à limiter l'apparition de condensation est relativement efficace, mais uniquement pendant une courte période d'utilisation. En effet, la pièce à main ne peut être utilisée de façon prolongée car la température de l'air contenu dans l'espace clos baisserait significativement favorisant ainsi l'apparition de condensation sur les fenêtres. En effet, en dessous d'une température de l'air contenu dans l'espace clos entre les deux fenêtres, typiquement pour des températures de l'air inférieures à environ 15°C, la formation de condensation sur les fenêtres est fortement favorisée.

### Objectifs de l'invention

La présente invention a pour objectif de proposer une nouvelle pièce à main refroidie pour la réalisation de traitement dermatologique par irradiation au moyen d'un faisceau lumineux. Cette nouvelle pièce à main permet avantageusement, même en cas d'utilisation prolongée, de limiter fortement l'apparition de condensation sur la fenêtre, qui est refroidie et destinée à venir au contact de la peau du patient.

### Résumé de l'invention

La pièce à main de l'invention comprend les caractéristiques suivantes connues du document US 2002/0120315 : elle comporte une première et une deuxième fenêtres et des moyens de refroidissement de la première fenêtre, lesdites première et deuxième fenêtres étant disposées en vis-à-vis l'une de l'autre et séparées par un espace contenant un gaz, et étant aptes à transmettre un faisceau lumineux, ladite première fenêtre étant destinée à être appliquée au contact de la peau lors du traitement.

De manière caractéristique selon l'invention, la pièce à main comporte des moyens de chauffage du gaz entre les deux fenêtres.

De préférence, mais de manière facultative selon l'invention, la pièce à main comporte les caractéristiques techniques additionnelles ci-après, prises isolément ou en combinaison :
- l'espace (E) entre les deux fenêtres est fermé ;
- le gaz entre les deux fenêtres est de l'air ;
- le gaz entre les deux fenêtres est de l'azote ou un mélange d'air et d'azote ;
- lesdits moyens de chauffage permettent de porter et de maintenir le gaz entre les deux fenêtres à une température supérieure au point de rosée du gaz ;
- lesdits moyens de chauffage permettent un chauffage de la deuxième fenêtre ;
- lesdits moyens de chauffage permettent de porter et de maintenir la deuxième fenêtre au dessus d'une température minimum (T2) ;
- ladite température minimum (T2) est supérieure à 15°C ;
- les premiers moyens de refroidissement permettent de refroidir et maintenir la première fenêtre à une température (T1) inférieure à 8°C ;
- les premiers moyens de refroidissement et les moyens de chauffage comportent un refroidisseur thermoélectrique à effet Peltier, et comportant une face chaude et une face froide, la face froide permettant le refroidissement de la première fenêtre et la face chaude permettant le chauffage de la deuxième fenêtre ;
- les premiers moyens de refroidissement et les moyens de chauffage sont en tout ou partie logés dans l'espace (E) entre les deux fenêtres ;
- le refroidisseur thermoélectrique à effet Peltier est logé entre les deux fenêtres ;
- le refroidisseur thermoélectrique à effet Peltier a la forme d'un anneau ;
- les moyens chauffage comportent un élément thermiquement conducteur, qui est positionné au contact de la face intérieure de la deuxième fenêtre et de la face chaude du refroidisseur thermoélectrique à effet Peltier, et qui permet de conduire jusqu'à la deuxième fenêtre une partie de la chaleur délivrée par la face chaude du refroidisseur thermoélectrique à effet Peltier, et d'évacuer à l'extérieur de l'espace (E) une partie de la chaleur délivrée par la face chaude du refroidisseur thermoélectrique à effet Peltier ;
- la pièce à main comporte des deuxièmes moyens de refroidissement de l'élément thermiquement conducteur ;
- les deuxièmes moyens de refroidissement comportent un refroidisseur thermoélectrique à effet Peltier et des moyens de dissipation de la chaleur de la face chaude de ce refroidisseur thermoélectrique ;
- les moyens de chauffage sont positionnés à l'extérieur de l'espace (E) entre les deux fenêtres ;
- les moyens de chauffage comportent une source chaude et un élément thermiquement conducteur (S") reliant thermiquement la source chaude à la deuxième fenêtre ;
- cet élément thermiquement conducteur (S") permet un chauffage par conduction de la face extérieure de la deuxième fenêtre (2) ;
- la pièce à main comporte des moyens de dissipation de la chaleur de la source chaude ;
- les premiers moyens de refroidissement comportent un élément (S') thermiquement conducteur en contact avec la première fenêtre et une source de froid pour le refroidissement de cet élément (S') ;
- la pièce à main comprend un refroidisseur thermoélectrique à effet Peltier dont la face froide constitue ladite source de froid, et dont la face chaude constitue ladite source chaude ;
- les éléments thermiquement conducteurs (S') et (S") sont séparés par un isolant thermique ;
- la pièce à main comporte un isolant thermique interposé entre la face interne de la deuxième fenêtre et les premiers moyens de refroidissement.

L'invention a également pour autre objet un appareil de traitement de la peau par irradiation au moyen d'un faisceau lumineux. De manière caractéristique selon l'invention, l'appareil comporte une pièce à main telle que précédemment décrite, une source lumineuse, et des moyens optiques pour la production à partir de la source lumineuse d'un faisceau lumineux traversant les première et deuxième fenêtres.

Plus particulièrement, la source lumineuse est une source laser. L'invention n'est toutefois pas limitée à ce type particulier de source lumineuse, mais s'étend à toute source lumineuse délivrant un rayonnement permettant de traiter la peau d'un patient. La source lumineuse peut par exemple comporter une ou plusieurs diodes électroluminescentes de forte puissance. Cette source lumineuse peut faire partie intégrante de la pièce à main ou être extérieure à la pièce à main en étant reliée à la pièce à main par une fibre optique.

Dans une variante particulière de réalisation, l'appareil comporte des moyens d'acquisition d'image, et les deux fenêtres de la pièce à main sont positionnées dans le champ de vision desdits moyens d'acquisition d'image.

### Brève description des figures

- la figure 1 est une représentation générale d'une pièce à main selon l'invention reliée à une source d'énergie lumineuse par une fibre optique,
- la figure 2 est une représentation en perspective d'un dispositif de refroidissement d'une pièce à main selon une première variante de l'invention,
- la figure 3 est une vue de côté du dispositif de refroidissement de la figure 2,
- la figure 4 est une vue en coupe longitudinale du dispositif de refroidissement des figures 2 et 3,
- la figure 5 est une représentation en perspective d'un dispositif de refroidissement d'une pièce à main selon une deuxième variante de l'invention
- la figure 6 est une vue de côté du dispositif de refroidissement de la figure 5
- la figure 7 est une vue en coupe longitudinale du dispositif de refroidissement des figures 5 et 6
- la figure 8 est un graphique représentant l'évolution des températures dans la pièce à main en fonction du temps et pour la première variante de l'invention.

### Description détaillée

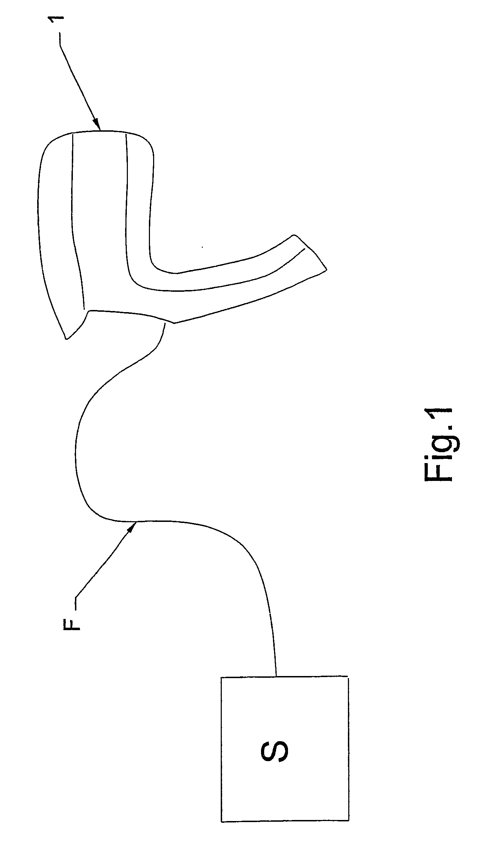

On a représenté à la figure 1 une pièce à main refroidie conforme à l'invention. Cette pièce à main est reliée par une fibre optique F à une source d'énergie lumineuse S, de préférence une source laser, et est utilisée pour le traitement par irradiation de la peau d'un patient, notamment pour un traitement vasculaire, pigmentaire ou dépilatoire. Dans une autre variante, la source lumineuse pourrait également être intégrée à la pièce à main, et il n'est pas nécessaire dans ce cas de mettre en oeuvre une fibre optique.

Pour atténuer la douleur ressentie par le patient, il est important de refroidir la peau du patient au niveau de la zone d'irradiation. Pour cette raison, cette pièce à main comporte un dispositif de refroidissement dont deux variantes de réalisation sont détaillées ci-après. Egalement, cette pièce à main permet avantageusement de limiter, voir d'éviter, l'apparition de condensation dans la pièce à main, découlant de ce refroidissement. On réduit ainsi avantageusement la formation de gouttes de condensation sur le trajet du faisceau lumineux thérapeutique, et le cas échéant dans le champ de vision de la caméra de la pièce à main, lorsque la pièce comporte une caméra pour effectuer une acquisition d'image de la zone traitée.

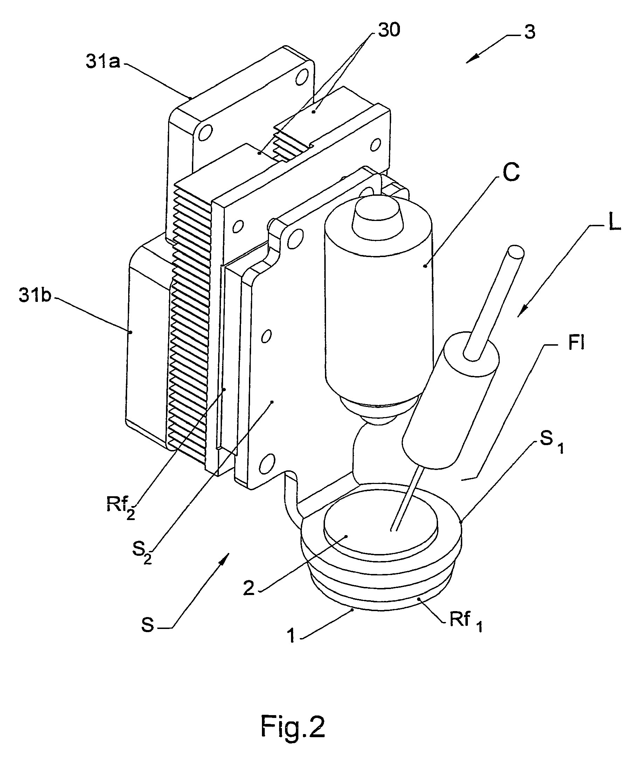

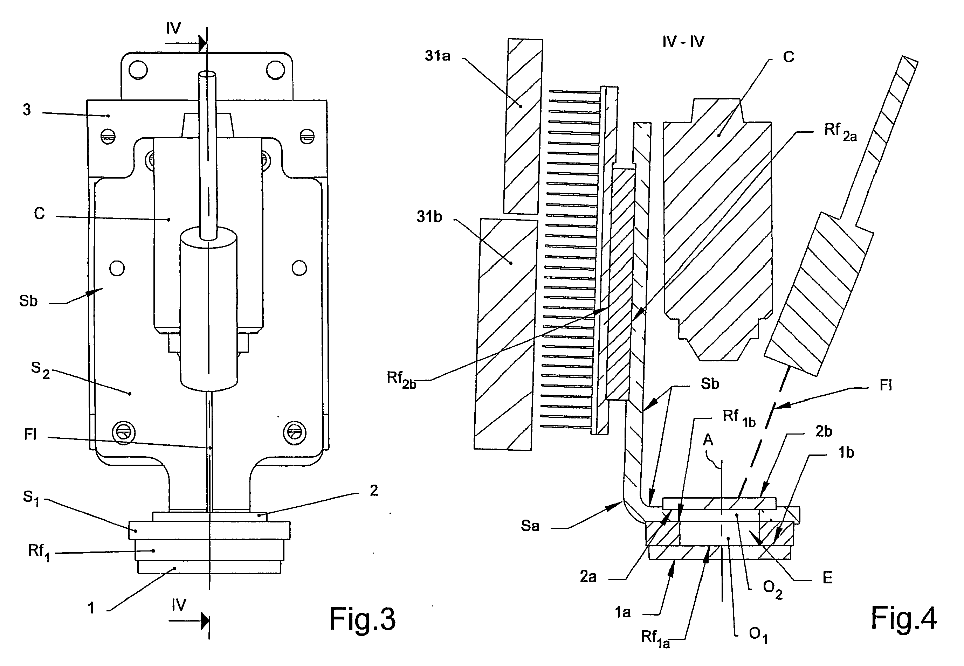

Les figures 2 à 4 représentent de manière simplifiée une pièce à main selon une première variante de l'invention équipée d'un premier moyen de refroidissement permettant de refroidir la fenêtre au contact de la peau du patient, d'un deuxième moyen de refroidissement et de moyens de chauffage. On a également représenté sur ces figures des moyens optiques L émettant un faisceau lumineux FI ainsi qu'un dispositif d'acquisition d'image, par exemple une caméra C, pour visualiser sur un écran (non représenté) la zone de traitement irradiée par le faisceau lumineux FI. Les moyens optiques L comprennent par exemple une pluralité de lentilles et de miroirs (non représentés) permettant de focaliser le faisceau lumineux FI.

La pièce à main de cette première variante comprend une première fenêtre 1, destinée à être traversée par le faisceau lumineux FI. Le matériau de cette première fenêtre 1 est ainsi choisi en sorte d'être transparent à la longueur d'onde ou pour une gamme de longueur d'ondes du faisceau lumineux FI. Par exemple, on choisira une fenêtre 1 en saphir, transparente pour une gamme de longueur d'ondes allant de 400nm à 3000nm, car ce matériau présente en outre une bonne conductivité thermique. La fenêtre 1 est l'élément qui est en contact direct avec la peau du patient lors d'un traitement réalisé avec la pièce à main. A cet effet, il convient de refroidir la fenêtre 1 de préférence avant, et pendant l'utilisation de la pièce à main sur la peau d'un patient.

La fenêtre 1 comprend une première face extérieure 1 a destinée à être au contact de la peau et une deuxième face intérieure 1b opposée à ladite première face 1 a.

Un premier moyen de refroidissement, par exemple un refroidisseur thermoélectrique Rf₁ à effet Peltier est fixé sur la face 1b de la première fenêtre 1 et est destiné à refroidir ladite fenêtre 1. Un tel refroidisseur est communément désigné par l'homme du métier TEC pour « thermoelectric cooler ». De manière avantageuse selon l'invention, le refroidisseur Rf₁ est un anneau comprenant une ouverture O₁ permettant le passage du faisceau lumineux FI et la visualisation de la zone de traitement au moyen de la caméra C.

L'anneau du refroidisseur Rf₁ comprend une première face Rf₁ₐ fixée sur la face 1b de la fenêtre 1 et une deuxième face Rf_{1b} opposée à ladite première face Rf₁ₐ. L'anneau étant un TEC destiné à refroidir la fenêtre 1 en saphir, sa face Rf₁ₐ constitue la face froide du moyen de refroidissement Rf₁ et sa face R_{1b} la face chaude.

La face chaude Rf_{1b} du refroidisseur Rf₁ est également fixée sur un élément thermiquement conducteur, en particulier un support S de fenêtre, comprenant une face Sa et une face Sb opposée à ladite face Sa. Le support S est par exemple réalisé en cuivre, et est disposé de manière à ce que l'anneau du refroidisseur Rf₁ soit pris en sandwich entre d'une part la face 1 b de la fenêtre 1 et d'autre part la face Sa du support S. Plus précisément, la face Sa du support S est fixée à la face Rf_{1b} du moyen de refroidissement Rf₁.

Le support S a sensiblement la forme d'un L présentant une embase S₁ et une paroi S₂ sensiblement perpendiculaire à ladite embase S₁. L'embase S₁, sur laquelle est fixé le moyen de refroidissement Rf₁ comprend une ouverture traversante O₂ alignée axialement selon un axe A avec l'ouverture O₁ de l'anneau du moyen de refroidissement Rf₁. Cette ouverture permet avantageusement le passage du faisceau lumineux FI et la visualisation de la zone de traitement au moyen de la caméra C.

Egalement, une seconde fenêtre 2 transparente à la longueur d'ondes ou dans la gamme de longueurs d'ondes du faisceau FI, est fixée sur l'embase S₁. Cette seconde fenêtre 2 est par exemple en verre. Cette seconde fenêtre 2 comprend une face intérieure 2a et une face extérieure 2b opposée à ladite face 2a. Plus particulièrement, la face 2a de la fenêtre 2 est fixée à la face Sb de l'embase S₁ du support de fenêtre S. En outre, la deuxième fenêtre 2 permet, comme l'embase S₁ du support S, l'anneau du refroidisseur Rf₁ et la fenêtre 1 en saphir, le passage du faisceau lumineux FI et la visualisation de la zone de traitement à l'aide de la caméra C.

Par ailleurs, la cavité délimitée par les deux fenêtres 1 et 2 par l'anneau refroidisseur Rf₁ et par le support S constitue un espace E, de préférence clos hermétiquement, et renfermant un gaz, par exemple à la pression atmosphérique. Le plus souvent ce gaz sera de l'air. Cependant, lorsque l'espace E est clos hermétiquement, on peut envisager de remplir cet espace E avec tout autre gaz, par exemple avec de l'azote ou un mélange d'air et d'azote.

De manière avantageuse selon l'invention, le moyen de refroidissement Rf₁ agit comme une pompe à chaleur en prenant des calories à une source froide (la fenêtre 1) pour les restituer à une source chaude (le support S). Ainsi, la fenêtre 1 se refroidit tandis que le support S et, par conduction la fenêtre 2, se réchauffent, ce qui permet d'abaisser la température de la fenêtre 1 au contact de la peau du patient et de chauffer le gaz renfermé dans l'espace E. Pour cette raison, les moyens de chauffage préalablement cités comprennent, dans cet exemple de réalisation de l'invention, la face chaude Rf_{1b} du refroidisseur Rf₁ et le support S. Lesdits moyens de chauffage sont destinés à porter la température de l'espace E à une température supérieure à la température de condensation du gaz compris dans l'espace E. Dans l'exemple particulier de réalisation de la première variante, les moyens de chauffage permettent de réchauffer l'espace E par un chauffage de la face intérieure 2a de la deuxième fenêtre 2.

En outre, la paroi S₂ du support S est reliée à un deuxième moyen de refroidissement, par exemple un refroidisseur thermoélectrique Rf₂ à effet Peltier (TEC). Ce refroidisseur Rf₂ a la forme d'une plaque rectangulaire qui permet d'éviter une augmentation trop forte de la température du support S. En effet, pour éviter l'apparition de condensation, il convient de maintenir le gaz compris dans l'espace E à une température supérieure au point de rosée du gaz contenu dans l'espace E. De manière connue, ce point de rosée dépend notamment de la pression du gaz, et du taux d'humidité relative (RH) du gaz. Pour de l'air à la pression atmosphérique et présentant un taux d'humidité relative (RH) maximum de 50%, ce point de rosée est toujours inférieur à 15°C. Lorsque le gaz est de l'air, on maintient de préférence le gaz à une température minimum supérieure à 15°C. Néanmoins, il est également préférable d'éviter une augmentation trop importante de la température de la deuxième fenêtre 2 au-dessus de cette limite (15°C dans le cas de l'air à la pression atmosphérique), afin de ne pas nuire au rendement du refroidissement de la première fenêtre 1.

Plus précisément, ce refroidisseur Rf₂ comprend une première face Rf₂ₐ, fixée contre la face Sa de la paroi S₂ du support S, et une deuxième face Rf_{2b} opposée à ladite première face Rf₂ₐ.

Le refroidisseur Rf₂ étant un TEC destiné à refroidir le support S, sa face Rf₂ₐ constitue la face froide dudit deuxième moyen de refroidissement Rf_{2b} et sa face Rf_{2b} la face chaude.

Avantageusement, et pour améliorer l'évacuation de la chaleur du support S et améliorer le rendement des moyens de refroidissement Rf₁ et Rf₂, le deuxième moyen de refroidissement Rf₂ est associé à des moyens de dissipation de la chaleur comprenant un radiateur 3, par exemple en cuivre, et des ailettes 30 associés à deux ventilateurs 31 a, 31 b pour accélérer l'évacuation de la chaleur.

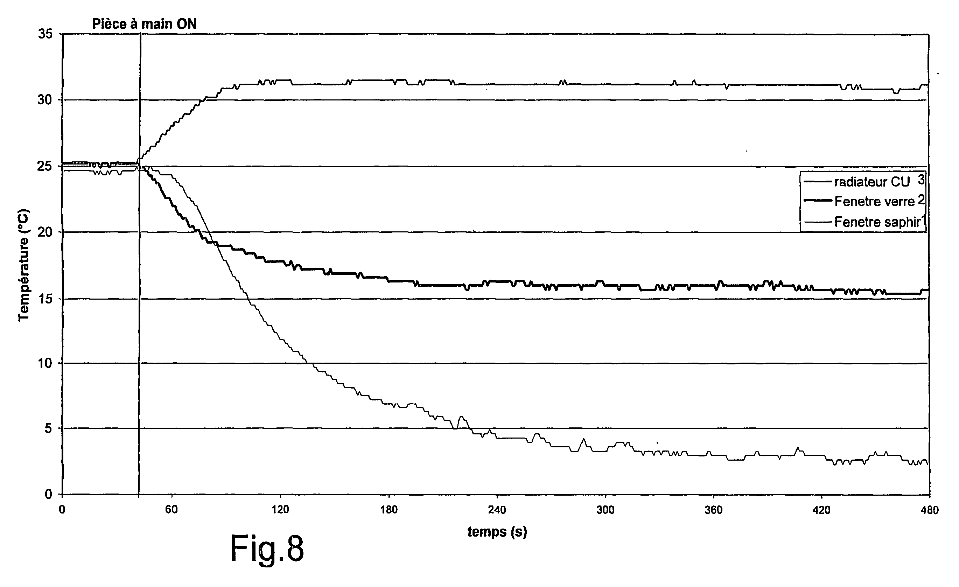

Dans un mode préféré d'utilisation de la pièce à main représenté à la figure 8, le refroidisseur Rf₁ opère afin de ramener et de maintenir rapidement la température (T1) de la fenêtre 1 en saphir à une température comprise entre 4°C et 8°C et le refroidisseur Rf₂ permet de maintenir la température du support S à une température comprise entre 15°C et 20°C. Le support S étant un élément thermiquement conducteur lié à la face chaude Rf_{1b} du refroidisseur Rf₁, la fenêtre 2 en verre voit également sa température (T2) ramenée et maintenue rapidement à une température comprise entre 15°C et 20°C.

En outre, l'effet Peltier ayant comme conséquence le transfert de chaleur d'une source froide vers une source chaude, le radiateur 3 voit sa température augmentée entre 30°C et 35°C.

L'objectif visé par la présente invention est d'empêcher l'apparition de condensation sur les fenêtres 1 et 2. Cet objectif est atteint en portant et en maintenant la température du gaz compris dans l'espace E à une température supérieure à la température de condensation du gaz.

L'utilisation simultanée de deux moyens de refroidissement séparés permet d'obtenir une meilleure efficacité et un meilleur contrôle des températures. En effet, alors que la face chaude Rf_{1b} du refroidisseur Rf₁ tend à réchauffer le support S par transfert de chaleur de la fenêtre 1 en saphir vers ledit support S, le deuxième moyen de refroidissement Rf₂ contribue à maintenir la température de la fenêtre 2 entre 15°C et 20°C en refroidissant de manière simultanée ledit support S.

Une pièce à main telle que décrite ci-dessus peut être obtenue au moyen d'un refroidisseur Rf₂ (TEC) standard de 40x40x4 mm d'une puissance de 75W et d'un refroidisseur Rf₁ annulaire de 26 mm de diamètre externe, de 14 mm de diamètre interne et d'une puissance de 6,6 W. Les résultats obtenus avec ces paramètres sont visibles sur le graphique de la figure 8 montrant l'évolution de la température du radiateur 3 et des fenêtres 1, 2 en fonction du temps (en secondes).

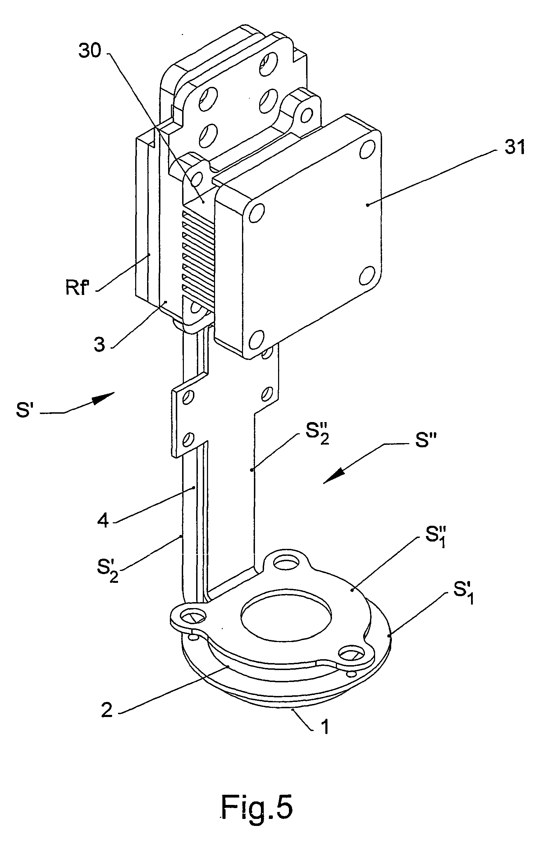

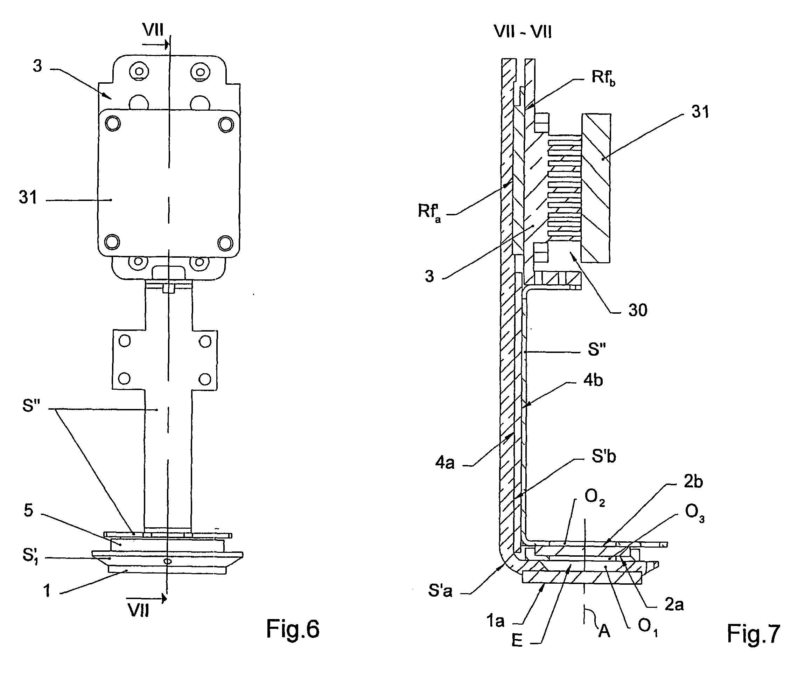

On a représenté aux figures 5 à 7, une pièce à main selon une deuxième variante de l'invention équipée de premiers moyens de refroidissement de la première fenêtre 1 et de moyen de chauffage de la deuxième fenêtre 2.

Plus particulièrement, dans cette deuxième variante de l'invention les premiers moyens de refroidissement comprennent par exemple un refroidisseur Rf thermoélectrique à effet Peltier, et sont associés à un premier élément thermiquement conducteur, en particulier un support de fenêtre S' pour la fenêtre 1, et à un deuxième élément thermiquement conducteur, en particulier un support de fenêtre S" pour la fenêtre 2, les deux supports étant par exemple réalisés en cuivre.

Le support S' comprend une face intérieure S'a sur laquelle est fixée la première fenêtre 1 destinée à être refroidie et une face extérieure S'b opposée à ladite face S'a. Plus particulièrement, le support S' a sensiblement la forme d'un L présentant une embase S'₁, comprenant comme dans la première variante une ouverture O₁, et une paroi S'₂ sensiblement perpendiculaire à ladite embase S'₁. La face extérieure 1b de la première fenêtre 1 est fixée à la face intérieure S'a de l'embase S'₁ du support S'.

Par ailleurs, le refroidisseur Rf comprend une première face Rf'ₐ, par laquelle il est fixé à la face S'b de la paroi S'₂ du support S', et une deuxième face Rf'_{b} opposée à la dite première face Rf'ₐ.

Le refroidisseur Rf étant un TEC destiné à refroidir la fenêtre 1 et chauffer la fenêtre 2, la face Rf'ₐ fixée sur le premier support S' constitue la face froide du refroidisseur Rf et la face Rf'_{b} la face chaude. Pour cette raison, on a fixé sur ladite face chaude Rf'_{b} du refroidisseur Rf des moyens de dissipation de la chaleur comprenant un radiateur 3 avec des ailettes 30 associés à un ventilateur 31 pour l'évacuation de la chaleur.

Le support S" est destiné à relier thermiquement le radiateur 3 à la fenêtre 2. Ledit support S" a sensiblement la forme d'un U et comprend une ouverture O₂ permettant le passage d'un faisceau lumineux FI et la visualisation de la zone de traitement au moyen d'une caméra.

Le support S" est également lié a une plaque d'isolation 4 comprenant une première face 4a, par laquelle elle est fixée à la face S'b de la paroi S'₂ du support S', et une deuxième face 4b, opposée à ladite première face 4a, sur laquelle le deuxième support S" est fixé. De cette manière, la plaque d'isolation 4 permet d'isoler thermiquement le support S' destiné à être refroidi du support S" destiné à être réchauffé.

La deuxième fenêtre 2 est dans cette variante fixée via sa face extérieure 2b au deuxième support S" de manière à ce qu'elle soit réchauffée par ledit support S". En outre, la fenêtre 2 est axialement alignée selon l'axe A avec l'ouverture O₂.

Le support de fenêtre S", sur lequel est fixé la deuxième fenêtre 2, est en contact direct avec le radiateur 3, qui est lui-même en contact avec la face chaude Rf'_{b} du refroidisseur Rf. A cet effet, lors du fonctionnement de la pièce à main, la deuxième fenêtre 2 et le support de fenêtre S" sont réchauffés, par conduction, par la face chaude Rf'_{b} du refroidisseur Rf et par le radiateur 3, alors que la première fenêtre 1 et le premier support S' sont refroidis. Pour cette raison, on a avantageusement positionné la plaque d'isolation 4 entre les supports S' et S" pour isoler le support S' refroidi du support S" réchauffé.

Egalement, la pièce à main de la deuxième variante de l'invention comprend un disque isolant 5 pris en sandwich entre la face 2a de la deuxième fenêtre 2 et la face S'a de l'embase S'₁ du premier support de fenêtre S'. Ce disque isolant 5 permet d'isoler thermiquement la fenêtre 2, qui est réchauffée, de la fenêtre 1 et du support S' qui sont refroidis. Le disque 5 comprend en outre une ouverture O₃, axialement aligné sur l'axe A, permettant le passage d'un faisceau lumineux FI et la visualisation de la zone de traitement par une caméra.

Par ailleurs, les ouvertures O₁ et O₃ délimitées par les fenêtres 1 et 2 constituent, comme dans la première variante un espace E, de préférence clos hermétiquement, renfermant de l'air ou tout autre gaz, par exemple de l'azote ou un mélange d'air et d'azote.

Avantageusement selon cette deuxième variante de l'invention, le refroidissement de la première fenêtre 1 est réalisé grâce au support S' et au refroidisseur Rf' et le chauffage de la deuxième fenêtre 2 est réalisé grâce à la face chaude Rf'_{b} du refroidisseur Rf, au radiateur 3 et au support S". En effet, dans cette variante, la fenêtre 1 est refroidie par l'intermédiaire du premier support de fenêtre S' et de la face froide Rf'ₐ des premiers moyens de refroidissement Rf' alors que la fenêtre 2 est réchauffée par l'intermédiaire de la face chaude Rf'_{b} du refroidisseur Rf, du radiateur 3 et du deuxième support de fenêtre S".

L'invention n'est pas limitée à l'exemple de réalisation des figures annexées. D'autres variantes de réalisation à la portée de l'homme du métier et couvertes par les revendications annexées peuvent être envisagées, sans pour autant sortir du cadre de l'invention. Par exemple, les moyens de refroidissement ne se limitent pas à l'utilisation de refroidisseur thermoélectrique, tout autre moyen de refroidissement connu de l'homme de métier pouvant également valablement être utilisé dans une pièce à main couverte par les revendications annexées.

De la même manière, les moyens de chauffage ne se limitent pas à l'utilisation d'un élément thermiquement conducteur en contact avec la face chaude du refroidisseur, mais peuvent être remplacés par tout moyen équivalent permettant un chauffage de la deuxième fenêtre 2 . Plus généralement encore, les moyens de chauffage de la deuxième fenêtre 2 peuvent être remplacés par tout moyen permettant de chauffer l'air contenu dan l'espace E entre les deux fenêtres 1 et 2, l'invention n'étant pas limitée à un chauffage de ce gaz par chauffage de la deuxième fenêtre.

Dans les variantes de réalisation des figures annexées, la pièce à main comporte un moyen d'acquisition d'image (camera) permettant au manipulateur de la pièce à main de visualiser la zone de traitement. Néanmoins, dans une variante de réalisation plus simple, la pièce à main peut ne pas être équipée de moyen d'acquisition d'image.

## Revendications

1. Pièce à main pour le traitement de la peau par irradiation au moyen d'un faisceau lumineux (FI), ladite pièce à main comportant une première (1) et une deuxième (2) fenêtres et des premiers moyens de refroidissement de la première fenêtre (1), lesdites première (1) et deuxième (2) fenêtres étant disposées en vis-à-vis l'une de l'autre et séparées par un espace (E) contenant un gaz, et étant aptes à transmettre un faisceau lumineux (FI), ladite première fenêtre (1) étant destinée à être appliquée au contact de la peau lors du traitement, **caractérisée en ce qu'**elle comporte des moyens de chauffage du gaz entre les deux fenêtres (1, 2).

2. Pièce à main selon la revendication 1, **caractérisée en ce que** l'espace (E) entre les deux fenêtres (1, 2) est fermé.

3. Pièce à main selon la revendication 1 ou 2, **caractérisée en ce que** le gaz entre les deux fenêtres (1, 2) est de l'air.

4. Pièce à main selon la revendication 1 ou 2, **caractérisée en ce que** le gaz entre les deux fenêtres (1, 2) est de l'azote ou un mélange d'air et d'azote.

5. Pièce à main selon l'une des revendications 1 à 4, **caractérisée en ce que** les lesdits moyens de chauffage permettent de porter et de maintenir le gaz entre les deux fenêtres (1, 2) à une température supérieure au point de rosée du gaz.

6. Pièce à main selon l'une des revendications 1 à 5, **caractérisée en ce que** lesdits moyens de chauffage sont agencés de manière à permettre un chauffage de la deuxième fenêtre (2).

7. Pièce à main selon la revendication 6, **caractérisée en ce que** les moyens de chauffage permettent de porter et de maintenir la deuxième fenêtre (2) au dessus d'une température minimum (T2).

8. Pièce à main selon la revendication 7, **caractérisée en ce que** la température minimum (T2) est supérieure à 15°C.

9. Pièce à main selon l'une des revendications 1 à 8, **caractérisée en ce que** les premiers moyens de refroidissement permettent de refroidir et maintenir la première fenêtre (1) à une température (T1) inférieure à 8°C.

10. Pièce à main selon l'une des revendications 1 à 9, **caractérisée en ce que** les premiers moyens de refroidissement et les moyens de chauffage comportent un refroidisseur thermoélectrique (Rf₁) à effet Peltier, et comportant une face chaude (Rf_{1b}) et une face froide (Rf₁ₐ), la face froide (Rf₁ₐ) permettant le refroidissement de la première fenêtre (1) et la face chaude (Rf_{1b}) permettant le chauffage de la deuxième fenêtre (2).

11. Pièce à main selon l'une des revendications 1 à 10, **caractérisée en ce que** les premiers moyens de refroidissement et les moyens de chauffage sont en tout ou partie logés dans l'espace (E) entre les deux fenêtres (1, 2).

12. Pièce à main selon les revendications 10 et 11, **caractérisée en ce que** le refroidisseur thermoélectrique à effet Peltier (Rf₁) est logé entre les deux fenêtres (1,2).

13. Pièce à main selon la revendication 12, **caractérisée en ce que** le refroidisseur thermoélectrique à effet Peltier (Rf₁) a la forme d'un anneau.

14. Pièce à main selon les revendications 12 ou 13, **caractérisée en ce que** les moyens chauffage comportent un élément (S) thermiquement conducteur, qui est positionné au contact de la face intérieure (2a) de la deuxième fenêtre (2) et de la face chaude (Rf_{1b}) du refroidisseur thermoélectrique à effet Peltier (Rf₁), et qui permet de conduire jusqu'à la deuxième fenêtre (2) une partie de la chaleur délivrée par la face chaude (Rf_{1b}) du refroidisseur thermoélectrique à effet Peltier (Rf₁), et d'évacuer à l'extérieur de l'espace (E) une partie de la chaleur délivrée par la face chaude (Rf_{1b}) du refroidisseur thermoélectrique à effet Peltier (Rf₁).

15. Pièce à main selon la revendication 14, **caractérisée en ce qu'**elle comporte des deuxièmes moyens de refroidissement de l'élément (S) thermiquement conducteur.

16. Pièce à main selon la revendication 15, **caractérisée en ce que** les deuxièmes moyens de refroidissement comportent un refroidisseur thermoélectrique à effet Peltier (Rf₂) et des moyens de dissipation de la chaleur (3, 30, 31a, 31b) de la face chaude (Rf_{2b}) de ce refroidisseur thermoélectrique (Rf₂).

17. Pièce à main selon l'une des revendications 1 à 10, **caractérisée en ce que** les moyens de chauffage sont positionnés à l'extérieur de l'espace (E) entre les deux fenêtres (1,2).

18. Pièce à main selon la revendication 17, **caractérisée en ce que** les moyens de chauffage comportent une source chaude et un élément (S") thermiquement conducteur reliant thermiquement la source chaude à la deuxième fenêtre (2).

19. Pièce à main selon la revendication 17, **caractérisée en ce que** les moyens de chauffage comportent une source chaude et un élément (S") thermiquement conducteur reliant thermiquement la source chaude à la deuxième fenêtre (2) et qui permet un chauffage par conduction de la face extérieure (2b) de la deuxième fenêtre (2).

20. Pièce à main selon la revendication 18 ou 19, **caractérisée en ce qu'**elle comporte des moyens de dissipation de la chaleur (3, 30, 31) de la source chaude.

21. Pièce à main selon l'une des revendications 17 à 20, **caractérisée en ce que** les premiers moyens de refroidissement comportent un élément (S') thermiquement conducteur en contact avec la première fenêtre (1) et une source de froid pour le refroidissement de cet élément (S').

22. Pièce à main selon la revendication 21 et l'une des revendications 18 à 20, **caractérisée en ce qu'**elle comprend un refroidisseur thermoélectrique à effet Peltier (Rf) dont la face froide (Rf'ₐ) constitue ladite source de froid, et dont la face chaude (Rf'_{b}) constitue ladite source chaude.

23. Pièce à main selon les revendications 18 et 21, **caractérisée en ce que** les éléments thermiquement conducteurs (S') et (S") sont séparés par un isolant thermique (4).

24. Pièce à main selon l'une des revendications 17 à 23, **caractérisée en ce qu'**elle comporte un isolant thermique (5) interposé entre la face interne (2a) de la deuxième fenêtre (2) et les premiers moyens de refroidissement.

25. Appareil de traitement de la peau par irradiation au moyen d'un faisceau lumineux (FI), **caractérisé en ce qu'**il comporte une pièce à main visée à l'une des revendications 1 à 24, une source lumineuse (S), et des moyens optiques pour la production à partir de la source lumineuse d'un faisceau lumineux (FI) traversant lesdites première et deuxième fenêtres.

26. Appareil selon la revendication 25 **caractérisé en ce que** la source lumineuse (S) est une source laser.

27. Appareil selon la revendication 25 ou 26, **caractérisé en ce qu'**il comporte des moyens d'acquisition d'image (C), et **en ce que** les deux fenêtres (1, 2) sont positionnées dans le champ de vision desdits moyens d'acquisition d'image.

## Claims

1. Handpiece for treating the skin by irradiation by means of a light beam (FI), said handpiece including a first (1) and a second (2) window and first cooling means of the first window (1), said first (1) and second (2) window being arranged opposite one another and separated by a space (E) containing a gas, and being suitable for letting a light beam (FI) pass through, said first window (1) being intended to make contact with the skin during treatment, **characterised in that** it includes means for heating the gas between the two windows (1, 2).

2. Handpiece according to claim 1, **characterised in that** the space (E) between the two windows (1, 2) is closed.

3. Handpiece according to claim 1 or 2, **characterised in that** the gas between the two windows (1, 2) is air.

4. Handpiece according to claim 1 or 2, **characterised in that** the gas between the two windows (1, 2) is nitrogen or a mixture of air and nitrogen.

5. Handpiece according to one of claims 1 to 4, **characterised in that** said heating means enable the gas to be held and maintained between the two windows (1, 2) at a temperature higher than the dew-point temperature of the gas.

6. Handpiece according to one of claims 1 to 5, **characterised in that** said heating means are arranged in such a manner as to enable the second window (2) to be heated.

7. Handpiece according to claim 6, **characterised in that** the heating means enable the second window (2) to be held and maintained above a minimum temperature (T2).

8. Handpiece according to claim 7, **characterised in that** the minimum temperature (T2) is higher than 15°C.

9. Handpiece according to one of claims 1 to 8, **characterised in that** the first cooling means enable the first window (1) to be cooled and maintained at a temperature (T1) lower than 8°C.

10. Handpiece according to one of claims 1 to 9, **characterised in that** the first cooling means and the heating means include a Peltier-effect thermoelectric cooler (Rf₁) and include a warm face (Rf_{1b}) and a cold face (Rf₁ₐ), the cold face (Rf₁ₐ) enabling the first window (1) to be cooled and the warm face (Rf_{1b}) enabling the second window (2) to be heated.

11. Handpiece according to one of claims 1 to 10, **characterised in that** the first cooling means and the heating means are fully or partly housed in the space (E) between the two windows (1, 2).

12. Handpiece according to claims 10 and 11, **characterised in that** the Peltier-effect thermoelectric cooler (RF₁) is housed between the two windows (1, 2).

13. Handpiece according to claim 12, **characterised in that** the Peltier-effect thermoelectric cooler (RF₁) has the shape of a ring.

14. Handpiece according to claims 12 or 13, **characterised in that** the heating means include a thermal transfer element (S), which is positioned in contact with the interior face (2a) of the second window (2) and the warm face (Rf_{1b}) of the Peltier-effect thermoelectric cooler (RF₁), and which enables part of the heat supplied by the warm face (Rf_{1b}) of the Peltier-effect thermoelectric cooler (RF₁) to be transferred to the second window (2), and part of the heat supplied by the warm face (Rf_{1b}) of the Peltier-effect thermoelectric cooler (RF₁) to be evacuated to the outside of the space (E).

15. Handpiece according to claim 14, **characterised in that** it includes second means for cooling the thermal transfer element (S).

16. Handpiece according to claim 15, **characterised in that** the second cooling means include a Peltier-effect thermoelectric cooler (RF₂) and means for dissipating the heat (3, 30, 31 a, 31 b) from the warm face (Rf_{2b}) of this thermoelectric cooler (RF₂).

17. Handpiece according to one of claims 1 to 10, **characterised in that** the heating means are positioned outside of the space (E) between the two windows (1, 2).

18. Handpiece according to claim 17, **characterised in that** the heating means include a heat source and a thermal transfer element (S"), thermally linking the heat source to the second window (2).

19. Handpiece according to claim 17, **characterised in that** the heating means include a heat source and a thermal transfer element (S"), thermally linking the heat source to the second window (2) and enabling the exterior face (2b) of the second window (2) to be heated by conduction.

20. Handpiece according to claim 18 or 19, **characterised in that** it includes means for dissipating the heat (3, 30, 31) from the heat source.

21. Handpiece according to one of claims 17 to 20, **characterised in that** the first cooling means include a thermal transfer element (S') in contact with the first window (1) and a cold source for the cooling of this element (S').

22. Handpiece according to claim 21 and one of claims 18 to 20, **characterised in that** it includes a Peltier-effect thermoelectric cooler (Rf'), the cold face (Rf'ₐ) of which constitutes said cold source, and the warm face (Rf'_{b}) of which constitutes said heat source.

23. Handpiece according to claims 18 and 21, **characterised in that** the thermal transfer elements (S') and (S") are separated by a thermal insulator (4).

24. Handpiece according to one of claims 17 to 23, **characterised in that** it includes a thermal insulator (5) arranged between the internal face (2a) of the second window (2) and the first cooling means.

25. Device for treating the skin by irradiation by means of a light beam (FI), **characterised in that** it includes a handpiece in accordance with one of claims 1 to 24, a light source (S) and optical means for producing from the light source a light beam (FI) passing through the first and second windows.

26. Device according to claim 25, **characterised in that** the light source (S) is a laser source.

27. Device according to claim 25 or 26, **characterised in that** it includes image acquisition means (C), and **in that** the two windows (1, 2) are positioned in the field of vision of said image acquisition means.

## Patentansprüche

1. Handstück zur Behandlung der Haut durch Bestrahlung mittels eines Lichtstrahls (Fl), wobei das Handstück ein erstes (1) und ein zweites Fenster (2) und erste Mittel zum Abkühlen des ersten Fensters (1) aufweist, wobei das erste (1) und das zweite Fenster (2) einander gegenüberliegend angeordnet und durch einen Gas enthaltenden Raum (E) voneinander getrennt sind und angepasst oder in der Lage sind, einen Lichtstrahl (Fl) zu übertragen, wobei das erste Fenster (1) angepasst oder dazu bestimmt ist, bei einer Behandlung in Kontakt mit der Haut gebracht zu werden, **dadurch gekennzeichnet, dass** es Mittel zum Erhitzen von Gas zwischen den beiden Fenstern (1, 2) aufweist.

2. Handstück nach Anspruch 1, **dadurch gekennzeichnet, dass** der Raum (E) zwischen den beiden Fenstern (1, 2) geschlossen ist.

3. Handstück nach Anspruch 1 oder 2, **dadurch gekennzeichnet, dass** das Gas zwischen den beiden Fenstern (1, 2) Luft ist.

4. Handstück nach Anspruch 1 oder 2, **dadurch gekennzeichnet, dass** das Gas zwischen den beiden Fenstern (1, 2) Stickstoff oder ein Gemisch aus Luft und Stickstoff ist.

5. Handstück nach einem der Ansprüche 1 bis 4, **dadurch gekennzeichnet, dass** die Heizmittel gestatten, das Gas zwischen den beiden Fenstern (1, 2) auf eine Temperatur zu bringen und auf dieser zu halten, die höher als der Taupunkt des Gases ist.

6. Handstück nach einem der Ansprüche 1 bis 5, **dadurch gekennzeichnet, dass** die Heizmittel so angeordnet sind, dass sie ein Erhitzen des zweiten Fensters (2) gestatten.

7. Handstück nach Anspruch 6, **dadurch gekennzeichnet, dass** die Heizmittel gestatten, das zweite Fenster (2) auf eine Temperatur zu bringen und auf dieser zu halten, die über einer Mindesttemperatur (T2) liegt.

8. Handstück nach Anspruch 7, **dadurch gekennzeichnet, dass** die Mindesttemperatur (T2) höher als 15°C ist.

9. Handstück nach einem der Ansprüche 1 bis 8, **dadurch gekennzeichnet, dass** die ersten Kühlmittel gestatten, das erste Fenster (1) auf eine Temperatur (T1) abzukühlen und auf dieser zu halten, die niedriger als 8°C ist.

10. Handstück nach einem der Ansprüche 1 bis 9, **dadurch gekennzeichnet, dass** die ersten Kühlmittel und die Heizmittel einen thermoelektrischen Kühler (Rf₁) mit Peltier-Effekt aufweisen, der eine heiße Seite (Rf_{1b}) und eine kalte Seite (Rf₁ₐ) aufweist, wobei die kalte Seite (Rf₁ₐ) das Abkühlen des ersten Fensters (1) und die heiße Seite (Rf_{1b}) das Erhitzen des zweiten Fensters (2) gestattet.

11. Handstück nach einem der Ansprüche 1 bis 10, **dadurch gekennzeichnet, dass** die ersten Kühlmittel und die Heizmittel ganz oder teilweise in dem Raum (E) zwischen den beiden Fenstern (1, 2) aufgenommen sind.

12. Handstück nach den Ansprüchen 10 und 11, **dadurch gekennzeichnet, dass** der thermoelektrische Kühler (Rf₁) mit Peltier-Effekt zwischen den beiden Fenstern (1, 2) aufgenommen ist.

13. Handstück nach Anspruch 12, **dadurch gekennzeichnet, dass** der thermoelektrische Kühler (Rf₁) mit Peltier-Effektin Form eines Rings vorliegt oder ausgebildet ist.

14. Handstück nach den Ansprüchen 12 oder 13, **dadurch gekennzeichnet, dass** die Heizmittel ein wärmeleitendes Element (S) aufweisen, das in Kontakt mit der Innenseite (2a) des zweiten Fensters (2) und mit der heißen Seite (Rf_{1b}) des thermoelektrischen Kühlers (Rf₁) mit Peltier-Effekt positioniert ist und gestattet, einen Teil der von der heißen Seite (Rf_{1b}) des thermoelektrischen Kühlers (Rf₁) mit Peltier-Effekt abgegebenen Wärme (bis) zum zweiten Fenster (2) zu leiten und einen Teil der von der heißen Seite (Rf_{1b}) des thermoelektrischen Kühlers (Rf₁) mit Peltier-Effekt abgegebenen Wärme nach außerhalb des Raums (E) abzuführen.

15. Handstück nach Anspruch 14, **dadurch gekennzeichnet, dass** es zweite Kühlmittel zum Abkühlen des wärmeleitenden Elements (S) aufweist.

16. Handstück nach Anspruch 15, **dadurch gekennzeichnet, dass** die zweiten Kühlmittel einen thermoelektrischen Kühler (Rf₂) mit Peltier-Effekt und Mittel zum Ableiten der Wärme (3, 30, 31a, 31b) von der heißen Seite (Rf_{2b}) dieses thermoelektrischen Kühlers (Rf₂) aufweisen.

17. Handstück nach einem der Ansprüche 1 bis 10, **dadurch gekennzeichnet, dass** die Heizmittel außerhalb des Raums (E) zwischen den beiden Fenstern (1, 2) angeordnet sind.

18. Handstück nach Anspruch 17, **dadurch gekennzeichnet, dass** Heizmittel eine heiße Quelle und ein wärmeleitendes Element (S") aufweisen, welches die heiße Quelle thermisch mit dem zweiten Fenster (2) verbindet.

19. Handstück nach Anspruch 17, **dadurch gekennzeichnet, dass** die Heizmittel eine heiße Quelle und ein wärmeleitendes Element (S") aufweisen, welches die heiße Quelle thermisch mit dem zweiten Fenster (2) verbindet und ein konduktives (leitendes) Erwärmen der Außenseite (2b) des zweiten Fensters (2) gestattet.

20. Handstück nach Anspruch 18 oder 19, **dadurch gekennzeichnet, dass** es Mittel zum Ableiten der Wärme (3, 30, 31) der heißen Quelle aufweist.

21. Handstück nach einem der Ansprüche 17 bis 20, **dadurch gekennzeichnet, dass** die ersten Kühlmittel ein wärmeleitendes Element (S') in Kontakt mit dem ersten Fenster (1) und eine Kältequelle für das Abkühlen dieses Elements (S') aufweisen.

22. Handstück nach Anspruch 21 und einem der Ansprüche 18 bis 20, **dadurch gekennzeichnet, dass** es einen thermoelektrischen Kühler (Rf') mit Peltier-Effekt aufweist, dessen kalte Seite (Rf'ₐ) die Kältequelle bildet und dessen heiße Seite (Rf'_{b}) die heiße Quelle bildet.

23. Handstück nach den Ansprüchen 18 und 21, **dadurch gekennzeichnet, dass** die wärmeleitenden Elemente (S' und S") durch einen Wärmedämmstoff (4) getrennt sind.

24. Handstück nach einem der Ansprüche 17 bis 23, **dadurch gekennzeichnet, dass** es einen Wärmedämmstoff (5) aufweist, der zwischen der Innenseite (2a) des zweiten Fensters (2) und dem/den ersten Kühlmittel(n) eingesetzt ist.

25. Gerät zum Behandeln der Haut durch Bestrahlung mittels eines Lichtstrahls (Fl), **dadurch gekennzeichnet, dass** es ein Handstück nach einem der Ansprüche 1 bis 24, eine Lichtquelle (S) und optische Mittel aufweist, um ausgehend von der Lichtquelle einen Lichtstrahl (Fl) zu erzeugen, der durch das erste und das zweite Fenster tritt.

26. Gerät nach Anspruch 25, **dadurch gekennzeichnet, dass** die Lichtquelle (S) eine Laserquelle ist.

27. Gerät nach Anspruch 25 oder 26, **dadurch gekennzeichnet, dass** es ein Mittel zur Bilderfassung (C) aufweist und dass die beiden Fenster (1, 2) in dem Sichtfeld der Bilderfassungsmittel positioniert sind.
